# EUROPEAN PATENT APPLICATION

(11) **EP 4 272 697 A1**
(43) Date of publication of application: **08.11.2023**
(21) Application number: 21914721.2
(22) Date of filing: 31.12.2021
(51) Int. Cl.: A61C 19/04, G01B 11/25

(54) **THREE-DIMENSIONAL SCANNING DEVICE, METHOD AND APPARATUS, STORAGE MEDIUM AND PROCESSOR**

(30) Priority: 31.12.2020 CN 202011640685; 31.12.2020 CN 202011642145
(71) Applicant: Shining 3D Tech Co., Ltd., Hangzhou, Zhejiang 311258 (CN)
(72) Inventor: MA, Chao, Hangzhou, Zhejiang 311258 (CN); ZHAO, Xiaobo, Hangzhou, Zhejiang 311258 (CN)
(74) Representative: Grünecker Patent- und Rechtsanwälte PartG mbB
(86) International application number: PCT/CN2021/143723
(87) International publication number: WO 2022/143992

(57) **Abstract**

The present disclosure discloses a three-dimensional scanning device, method and apparatus, a storage medium and a processor. The three-dimensional scanning device includes a projection device (10), configured to project a fringe-encoded image to a to-be-scanned object, wherein the fringe-encoded image includes a first fringe group; and a camera (12), configured to collect the to-be-scanned object to obtain a camera image, wherein the camera image is an image of the to-be-scanned object on an imaging plane of the camera, and the imaging plane includes a first imaging interval. When the to-be-scanned object is located within an effective depth-of-field (ΔL) range of the three-dimensional scanning device, an image of the first fringe group on the imaging plane is located within the first imaging interval, and only the first fringe group exists in the first imaging interval, and solve the technical problem that in the related art, the multiple image sequences are required to be complexly encoded to generate the structured light encoding pattern.

## Description

### Cross-Reference to Related Application

The present application claims priority to Chinese Patent Application No.2020116406858, entitled "THREE-DIMENSIONAL SCANNING METHOD AND APPARATUS, STORAGE MEDIUM AND PROCESSOR", and filed to the China National Intellectual Property Administration on December 31, 2020, and Chinese Patent Application No.2020116421453, entitled "THREE-DIMENSIONAL SCANNING APPARATUS AND METHOD", and filed to the China National Intellectual Property Administration on December 31, 2020, the entire contents of which are incorporated herein by reference.

### Technical Field

The present disclosure relates to the field of three-dimensional scanning, and in particular relates to a three-dimensional scanning device, method and apparatus, a storage medium and a processor.

### Background

In the related art, the means for acquiring dental cast data internationally in the field of dental diagnosis and treatment has gradually turned to the intraoral three-dimensional scanning technology from impression-based three-dimensional scanning. This technology is another revolution in digital processing of teeth. This technology abandons a dental cast data acquiring manner via impression, cast making, and three-dimensional scanning, and intraoral scanning can be directly performed to acquire tooth three-dimensional data. Two steps of impression and cast making are omitted in process time; material cost, labor cost and cast express fee needed in the above process are saved; and discomfort of customers during impression making can be avoided. The above advantages indicate that this technology is bound to be greatly developed, and has significant benefits on the market.

An oral cavity digital impression instrument is also called an intraoral three-dimensional scanner, and is a device which applies a probe type optical scanning head to directly scan an oral cavity of a patient and acquire three-dimensional shape and color texture information of surfaces of soft or hard tissues such as teeth, gums and mucosa in the oral cavity. A method for the device adopts an active structured light trigonometric survey imaging principle, and utilizes a digital projection system for projecting an active light pattern, and a camera acquisition system processes the acquired pattern through an algorithm for three-dimensional reconstruction and splicing.

When a structured light encoding pattern is designed, it is usually considered to decode the entire image by methods such as temporal phase unwrapping and spatial phase unwrapping. On the basis of obtaining a folding phase, phase unwrapping is also necessary to obtain a real absolute phase to solve the problem about periodicity of the folding phase. To globally unfold the phase, multiple image sequences or complex spatial encoding and decoding processes are usually required.

There is still no effective solution for the problems that in the related art, the multiple image sequences are required to be complexly encoded to generate the structured light encoding pattern.

### Summary

Embodiments of the present disclosure provide a three-dimensional scanning device, method and apparatus, a storage medium and a processor, so as to at least solve the technical problem that in the related art, multiple image sequences are required to be complexly encoded to generate a structured light encoding pattern.

According to one aspect of an embodiment of the present disclosure, a three-dimensional scanning device is provided, and includes a projection device, configured to project a fringe-encoded image to a to-be-scanned object, wherein the fringe-encoded image includes a first fringe group; and a camera, configured to collect the to-be-scanned object to obtain a camera image, wherein the camera image is an image of the to-be-scanned object on an imaging plane of the camera, the imaging plane includes a first imaging interval. When the to-be-scanned object is located within an effective depth-of-field range of the three-dimensional scanning device, an image of the first fringe group on the imaging plane is located within the first imaging interval, and only the first fringe group exists in the first imaging interval.

Optionally, a system included angle α is formed between a projection optical axis of the projection device and a collection optical axis of the camera. Optical parameters of the camera include lens magnification k₁, an effective depth of field ΔL of the three-dimensional scanning device and the first imaging interval di, and d₁=ΔL×tgα÷k₁.

Optionally, the projection device includes an image display element. The image display element includes a first display interval provided with the first fringe group. Optical parameters of the projection device include lens magnification k₂ of the projection device and the first display interval D₁, and D₁=ΔL×tgα÷k₂.

Optionally, the fringe-encoded image further includes a second fringe group adjacent to the first fringe group. The imaging plane includes a second imaging interval adjacent to the first imaging interval. When the to-be-scanned object is located within the effective depth-of-field range of the three-dimensional scanning device, an image of the second fringe group on the imaging plane is located within the second imaging interval, and only the second fringe group exists in the second imaging interval.

Optionally, the fringe-encoded image includes a plurality of fringe groups periodically arranged, and the first fringe groups and the second fringe groups are respectively located in a cycle.

Optionally, a system included angle α is formed between a projection optical axis of the projection device and a collection optical axis of the camera. Optical parameters of the camera include lens magnification k₁, an effective depth of field ΔL of the three-dimensional scanning device and the second imaging interval d₂, and d₂=ΔL×tgα÷k₁.

Optionally, the projection device includes an image display element. The image display element includes a second display interval provided with the second fringe group. Optical parameters of the projection device include lens magnification k₂ of the projection device and the second display interval D₂, and D₂=ΔL×tgα÷k₂.

Optionally, the device further includes a processor, configured to perform three-dimensional reconstruction on the to-be-scanned object based on the camera image.

Optionally, first imaging interval coordinates are preset in the processor; the processor determines, based on the camera image, pixel coordinates of a center of each fringe in the camera image; the processor determines, based on the pixel coordinates of the fringes and the first imaging interval coordinates, a number of each fringe in the camera image; and the processor performs, based on the pixel coordinates of the center of each fringe and the number of each fringe, three-dimensional reconstruction to obtain a three-dimensional digital model of the to-be-scanned object.

Optionally, a light plane of each fringe and a corresponding number thereof in the fringe-encoded image are preset in the processor; the processor determines, based on consistency between a number of each fringe in the camera image and the corresponding number of the light plane of each fringe, a light plane corresponding to the pixel coordinates of the center of each fringe; and the processor performs, based on the pixel coordinates of the center of each fringe and the corresponding light plane, trigonometric calculation to reconstruct a three-dimensional digital model of the to-be-scanned object.

According to another aspect of this embodiment of the present disclosure, a three-dimensional scanning method is further provided and is executed based on the above three-dimensional scanning device. The three-dimensional scanning device further includes a processor. The processor is configured to perform, based on the camera image, three-dimensional reconstruction on the to-be-scanned object. The three-dimensional scanning method includes following steps: projecting, by the projection device, the fringe-encoded image to the to-be-scanned object; collecting the to-be-scanned object by the camera to obtain the camera image, wherein the camera image is the image of the to-be-scanned object on the imaging plane of the camera, the imaging plane includes the first imaging interval, and when the to-be-scanned object is located within an effective depth-of-field range of the three-dimensional scanning device, the image of the first fringe group on the imaging plane is located within the first imaging interval, and only the first fringe group exists in the first imaging interval; and performing, by the processor, three-dimensional reconstruction on the to-be-scanned object based on the camera image.

Optionally, the three-dimensional scanning method further includes: determining, based on the camera image, pixel coordinates of a center of each fringe in the camera image; presetting first imaging interval coordinates in the processor, and determining a number of each fringe based on the pixel coordinates of the fringes and the first imaging interval coordinates; and performing three-dimensional reconstruction on the pixel coordinates of the center of each fringe based on the numbers to obtain a three-dimensional digital model of the to-be-scanned object.

According to another aspect of this embodiment of the present disclosure, a three-dimensional scanning method is further provided, and includes: projecting a fringe-encoded image to a to-be-scanned object, wherein the fringe-encoded image includes a time-encoded image or color-encoded image, the time-encoded image includes a plurality of time fringe patterns arranged based on time, and the color-encoded image includes a color fringe pattern encoded by a plurality of colors; collecting a three-dimensional reconstructed image of the to-be-scanned object, wherein a surface of the to-be-scanned object in the three-dimensional reconstructed image has the fringe-encoded image; and reconstructing, based on the three-dimensional reconstructed image, a three-dimensional model of the to-be-scanned object.

Optionally, when the fringe-encoded image is the time-encoded image, the three-dimensional scanning method includes: projecting a first time fringe pattern to the surface of the to-be-scanned object at the first time; obtaining a first time fringe image on the surface of the to-be-scanned object; projecting a second time fringe pattern to the surface of the to-be-scanned object at the second time; obtaining a second time fringe image on the surface of the to-be-scanned object; and determining a time image encoding table based on the first time fringe image and the second time fringe image.

Optionally, determining the time image encoding table based on the first time fringe image and the second time fringe image includes: determining a first encoding table based on the first time fringe image; determining a second encoding table based on the second time fringe image; and constructing the time image encoding table based on the first encoding table and the second encoding table.

Optionally, determining the first encoding table based on the first time fringe image includes: correspondingly assigning first encoded values to pixels with fringes in the first time fringe image, correspondingly assigning second encoded values to pixels without fringes in the first time fringe image, and constructing the first encoding table by the first encoded values and the second encoded values based on pixel position distribution of the first time fringe image; determining the second encoding table based on the second time fringe image includes: correspondingly assigning first encoded values to pixels with fringes in the second time fringe image, correspondingly assigning second encoded values to pixels without fringes in the second time fringe image, and constructing the second encoding table by the first encoded values and the second encoded values based on pixel position distribution of the second time fringe image; and constructing the time image encoding table based on the first encoding table and the second encoding table includes: arranging the encoded values at same pixel positions in the first encoding table and the second encoding table according to an obtaining sequence of the first time fringe image and the second time fringe image to serve as encoding sequences of corresponding pixels, and constituting the time image encoding table based on the encoding sequences.

Optionally, after obtaining the second time fringe image on the surface of the to-be-scanned object, the three-dimensional scanning method further includes: projecting a third time fringe pattern to the surface of the to-be-scanned object at the third time; obtaining a third time fringe image on the surface of the to-be-scanned object; and determining a time image encoding table based on the first time fringe image, the second time fringe image and the third time fringe image.

Optionally, determining the time image encoding table based on the first time fringe image, the second time fringe image and the third time fringe image includes: correspondingly assigning first encoded values to pixels with fringes in the first time fringe image, correspondingly assigning second encoded values to pixels without fringes in the first time fringe image, and constructing a first encoding table by the first encoded values and the second encoded values based on pixel position distribution of the first time fringe image; correspondingly assigning first encoded values to pixels with fringes in the second time fringe image, correspondingly assigning second encoded values to pixels without fringes in the second time fringe image, and constructing a second encoding table by the first encoded values and the second encoded values based on pixel position distribution of the second time fringe image; correspondingly assigning first encoded values to pixels with fringes in the third time fringe image, correspondingly assigning second encoded values to pixels without fringes in the third time fringe image, and constructing a third encoding table by the first encoded values and the second encoded values based on pixel position distribution of the third time fringe image; and arranging the encoded values at same pixel positions in the first encoding table, the second encoding table and the third encoding table according to an obtaining sequence of the first time fringe image, the second time fringe image and the third time fringe image to serve as encoding sequences of corresponding pixels, and constituting a time image encoding table based on the encoding sequences.

Optionally, encoding table adopts binary encoding, and encoded values corresponding to pixels with fringes in the time-encoded image are denoted by 1, and encoded values corresponding to pixels without fringes in the time-encoded image are denoted by 0.

Optionally, after determining a time image encoding table based on the first time fringe image and the second time fringe image, the three-dimensional scanning method further includes: projecting a fourth time fringe pattern to the surface of the to-be-scanned object to obtain a fourth time fringe image on the surface of the to-be-scanned object, and determining a sequence of each fringe in the fourth time fringe image based on the time image encoding table; and projecting a fifth time fringe pattern to the surface of the to-be-scanned object to obtain a fifth time fringe image on the surface of the to-be-scanned object, and determining a sequence of each fringe in the fifth time fringe image based on the time image encoding table, wherein the fifth time fringe pattern is obtained by deflecting each fringe in the fourth time fringe pattern by a distance d in a same direction.

Optionally, when the fringe-encoded image is the color-encoded image, the three-dimensional scanning method includes: projecting the color-encoded image to the surface of the to-be-scanned object, wherein the color-encoded image includes a first color fringe pattern and a second color fringe pattern; obtaining color fringe images on the surface of the to-be-scanned object, wherein the color fringe images include a first color fringe image and a second color fringe image; and determining a color image encoding table based on the first color fringe image and the second color fringe image.

Optionally, determining a color image encoding table based on the color fringe images includes: determining a first color encoding table based on the first color fringe image; determining a second color encoding table based on the second color fringe image; constructing the color image encoding table based on the first color encoding table and the second color encoding table.

Optionally, determining the first color encoding table based on the first color fringe image includes: correspondingly assigning a first encoding sequence to pixels with a first color in the first color fringe image, correspondingly assigning a fourth encoding sequence to pixels without the first color in the first color fringe image, and constructing the first color encoding table by the first encoding sequence and the fourth encoding sequence based on pixel position distribution of the first color fringe image; determining the second color encoding table based on the second color fringe image includes: correspondingly assigning a second encoding sequence to pixels with a second color in the second color fringe image, correspondingly assigning a fourth encoding sequence to pixels without the second color in the second color fringe image, and constructing the second color encoding table by the second encoding sequence and the fourth encoded sequence based on pixel position distribution of the second color fringe image; and constructing the color image encoding table based on the first color encoding table and the second color encoding table includes: superposing encoding sequence at same pixel positions in the first color encoding table and the second color encoding table as encoding sequences of corresponding pixels, and constituting the color image encoding table based on superimposed encoding sequences corresponding to a distribution of each pixel.

Optionally, encoding table adopts binary encoding, and a first encoding sequence corresponding to pixels with a first color in the color-encoded image is (0,0,1), and a second encoding sequence corresponding to pixels with a second color in the color-encoded image is (0,1,0), and a fourth encoding sequence corresponding to pixels without color in the color-encoded image is (0,0,0).

According to another aspect of this embodiment of the present disclosure, a three-dimensional scanning apparatus is further provided, and includes: a projection unit, configured to project a fringe-encoded image to a to-be-scanned object, wherein the fringe-encoded image includes a time-encoded image or color-encoded image, the time-encoded image includes a plurality of time fringe patterns arranged based on time, and the color-encoded image includes a color fringe pattern encoded by a plurality of colors; an acquisition unit, configured to collect a three-dimensional reconstructed image of the to-be-scanned object, wherein a surface of the to-be-scanned object in the three-dimensional reconstructed image has the fringe-encoded image; and a reconstruction unit, configured to reconstruct, based on the three-dimensional reconstructed image, a three-dimensional model of the to-be-scanned object.

According to another aspect of this embodiment of the present disclosure, a computer-readable storage medium is further provided and includes stored programs. The programs, when running, control a device where the computer-readable storage medium is located to execute the above three-dimensional scanning method.

According to another aspect of this embodiment of the present disclosure, a processor is further provided. The processor is configured to run programs. The programs, when running, execute the above three-dimensional scanning method.

In this embodiment of the present disclosure, the projection device is configured to project the fringe-encoded image to the to-be-scanned object, wherein the fringe-encoded image includes the first fringe group. The camera is configured to collect the to-be-scanned object to obtain the camera image, wherein the camera image is the image of the to-be-scanned object on the imaging plane of the camera, and the imaging plane includes the first imaging interval. When the to-be-scanned object is located within the effective depth-of-field range of the three-dimensional scanning apparatus, the image of the first fringe group on the imaging plane is located within the first imaging interval, and only the first fringe group exists in the first imaging interval. The three-dimensional scanning apparatus restricts, according to the linear propagation characteristic of light, the fringe-encoded image within the first imaging interval defined by a hardware structure of the three-dimensional scanning apparatus. Thus, by utilizing the first imaging interval as an encoding cycle and ensuring unique encoding of the fringe-encoded image in the encoding cycle, the unique encoding can be guaranteed by utilizing a small amount of encoding information (i.e., fewer sequence images or less space codes) of the fringe-encoded image. Accordingly, the three-dimensional scanning apparatus can be used by combining optical characteristics without relying on a high-difficulty hardware level, and dynamic scanning speed may also be increased by fewer image sequences or a simple space encoding and decoding method, thereby realizing a technical effect of improving the scanning efficiency, and then solving the technical problem that in the related art, the multiple image sequences are required to be complexly encoded to generate the structured light encoding pattern.

### Brief Description of the Drawings

Drawings illustrated herein are used to provide further understanding for the present disclosure and constitute a part of the present application. Exemplary embodiments of the present disclosure and descriptions thereof are used for explaining the present disclosure but do not improperly limit the present disclosure. In the drawings:
FIG. 1 is a schematic diagram of a three-dimensional scanning device according to an embodiment of the present disclosure;
FIG. 2 is a schematic diagram of optical parameters of a lens according to an embodiment of the present disclosure;
FIG. 3a is a schematic diagram I of a first time fringe pattern according to an embodiment of the present disclosure;
FIG. 3b is a schematic diagram II of a second time fringe pattern according to an embodiment of the present disclosure;
FIG. 3c is a schematic diagram III of a third time fringe pattern according to an embodiment of the present disclosure;
FIG. 3d is a schematic diagram of an encoding table of a time-encoded image according to an embodiment of the present disclosure;
FIG. 4a is a schematic diagram of a color-encoded image according to an embodiment of the present disclosure;
FIG. 4b is a schematic diagram of an encoding table of a color-encoded image according to an embodiment of the present disclosure;
FIG. 5 is a flowchart I of a three-dimensional scanning method according to an embodiment of the present disclosure;
FIG. 6 is a flowchart II of a three-dimensional scanning method according to an embodiment of the present disclosure;
FIG. 7 is a schematic diagram of encoding occlusions according to an embodiment of the present disclosure;
FIG. 8 is a schematic diagram of reconstructed fringe offset according to an embodiment of the present disclosure; and
FIG. 9 is a schematic diagram of a three-dimensional scanning apparatus according to an embodiment of the present disclosure.

### Detailed Description of the Embodiments

For the purpose of making those skilled in the art better understand schemes of the present disclosure, technical schemes in embodiments of the present disclosure are clearly and completely described in conjunction with drawings in the embodiments of the present disclosure as below, and obviously, the ones described herein are merely a part of the embodiments of the present disclosure and not all the embodiments. Based on the embodiments of the present disclosure, all other embodiments obtained by those of ordinary skill in the art without creative work fall within the scope of protection of the present disclosure.

It needs to be explained that terms such as "first" and "second" in the description and claims of the present disclosure and the above drawings are used to distinguish similar objects but not necessarily used to describe specific sequences or precedence orders. It should be understood that adopted data can be exchanged under a proper situation so as to implement the embodiments, described herein, of the present disclosure in sequence except the illustrated or described sequences. In addition, terms "include" and "have" and any transformations thereof are intended to cover non-exclusive inclusion, for example, a process, a method, a system, a product or a device including a series of steps or units is not limited to clearly-listed steps or units, while may include unclearly-listed other steps or units or other inherent steps or units of the process, the method, the product or the device.

According to an embodiment of the present disclosure, an embodiment of a method for deflecting projection light rays based on three-dimensional scanning is provided. It needs to be explained that steps shown in a flowchart of the drawings may be performed in a computer system with a set of computer executable instructions. In addition, although a logical sequence is shown in the flowchart, the illustrated or described steps may be performed in sequence different from the sequence herein under some situations.

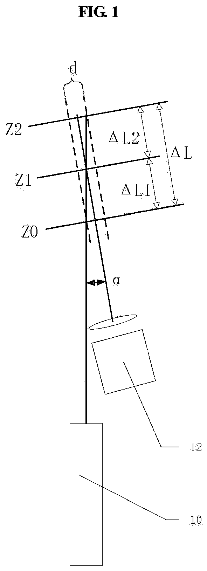

FIG. 1 is a schematic diagram of a three-dimensional scanning device according to an embodiment of the present disclosure. As shown in FIG. 1, the device includes a projection device 10, configured to project a fringe-encoded image to a to-be-scanned object, wherein the fringe-encoded image includes a first fringe group; and a camera 12, configured to collect the to-be-scanned object to obtain a camera image, wherein the camera image is an image of the to-be-scanned object on an imaging plane of the camera, and the imaging plane includes a first imaging interval. When the to-be-scanned object is located within an effective depth-of-field range of the three-dimensional scanning device, an image of the first fringe group on the imaging plane is located within the first imaging interval, and only the first fringe group exists in the first imaging interval.

In this embodiment of the present disclosure, the projection device is configured to project the fringe-encoded image to the to-be-scanned object, wherein the fringe-encoded image includes the first fringe group. The camera is configured to collect the to-be-scanned object to obtain the camera image, wherein the camera image is the image of the to-be-scanned object on the imaging plane of the camera, and the imaging plane includes the first imaging interval. When the to-be-scanned object is located within the effective depth-of-field range of the three-dimensional scanning device, the image of the first fringe group on the imaging plane is located within the first imaging interval, and only the first fringe group exists in the first imaging interval. The three-dimensional scanning device restricts, according to the linear propagation characteristic of light, the fringe-encoded image within the first imaging interval defined by a hardware structure of the three-dimensional scanning device. Thus, by utilizing the first imaging interval as an encoding cycle and ensuring unique encoding of the fringe-encoded image in the encoding cycle, the unique encoding can be guaranteed by utilizing a small amount of encoding information (i.e., fewer sequence images or less space codes) of the fringe-encoded image. Accordingly, the three-dimensional scanning device can be used by combining optical characteristics without relying on a high-difficulty hardware level, and dynamic scanning speed may also be increased by fewer image sequences or a simple space encoding and decoding method, thereby realizing a technical effect of improving the scanning efficiency, and then solving the technical problem that in the related art, the multiple image sequences are required to be complexly encoded to generate the structured light encoding pattern.

Optionally, as shown in FIG. 1, an effective depth of field ΔL is a distance between Z0 and Z2, wherein Z0 is a near-point position, Z2 is a far-point position, and when the to-be-scanned object is located between Z0 and Z2, a clear image of the to-be-scanned object can be collected by the camera.

That is, the effective depth of field ΔL=front depth of field ΔL1+rear depth of field ΔL2, wherein ΔL1+ΔL2 ranges from 10 mm to 20 mm.

Optionally, magnification of an optical system of the camera is usually about 3:1, and an imaging interval (e.g., the first imaging interval or a second imaging interval) of a fixed projection light ray on the camera image is d, namely a single-cycle range.

According to the technical solution claimed by the present application, in a scanning scenario with a small range of field of view, a structured light fringe pattern with a same encoded value inevitably moves in the image plane of the camera or the projection device within the effective range of depth of field due to an included angle of a binocular system and magnification of an optical lens, and the movement range is decided by three aspects: the effective depth of field, the included angle of the optical system and the magnification of the lens.

Optionally, the movement range includes a display interval of the projection device (e.g., a first display interval or a second display interval) and the imaging interval of the camera (e.g., the first imaging interval or the second imaging interval). After optical parameters of the projection device and the camera in the three-dimensional scanning device are determined, the movement range is determined, and by designing unique fringe encoding within the movement range, the unique encoded value across the entire image plane can be guaranteed. Due to the linear propagation characteristic of light, the light ray within the display interval cannot jump out of the imaging interval.

Optionally, the imaging movement range is utilized as one encoding cycle, unique encoding is guaranteed in the encoding cycle. Because the cycle can be ensured to be short according to optical design, the unique encoding can be guaranteed by utilizing a small amount of encoding information (fewer sequence images or less space codes).

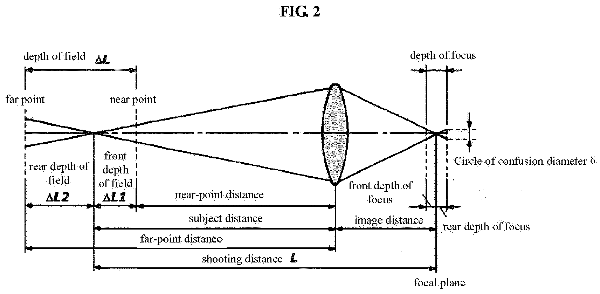

FIG. 2 is a schematic diagram of optical parameters of a lens according to an embodiment of the present disclosure. As shown in FIG. 2, the optical parameters include: a front depth of focus and a rear depth of focus obtained according to a focal plane of the lens, and positions of circles of confusion in front of and behind the focal plane; and an effective depth of field of the lens, wherein the effective depth of field of the lens includes a front depth of field determined based on the front depth of focus, a rear depth of field determined based on the rear depth of focus and a shooting distance between the location of an object point (i.e., the to-be-scanned object) and the lens. The shooting distance includes a subject distance between the lens and the to-be-scanned object, a near-point distance between a near point of the depth of field and the lens, and a far-point distance between a far point of the depth of field and the lens.

Optionally, the lens shown in FIG. 2 may be a lens of a camera or a lens of a projection device.

Optionally, when the lens shown in FIG. 2 is the lens of the camera, the to-be-scanned object may be arranged within the effective depth-of-field range of the camera, a collected camera image of the to-be-scanned object is arranged within the range of the depth of focus, and an imaging interval (e.g., a first imaging interval or a second imaging interval) may be calculated according to the optical parameters determined based on the lens of the camera.

Optionally, when the lens shown in FIG. 2 is the lens of the projection device, a negative (or a phase) of a fringe-encoded image may be arranged within the range of the depth of focus of the projection device, the to-be-scanned object is arranged within the effective depth-of-field range of the projection device, and a display interval (e.g., a first display interval or a second display interval) may be calculated according to the optical parameters determined based on the lens of the projection device.

As an optional embodiment, a system included angle α is formed between a projection optical axis of the projection device and a collection optical axis of the camera. Optical parameters of the camera include lens magnification k₁, an effective depth of field ΔL of the three-dimensional scanning device and a first imaging interval di, and d₁=ΔL×tgα÷k₁.

As an optional embodiment, the projection device includes an image display element. The image display element includes a first display interval provided with a first fringe group. Optical parameters of the projection device include lens magnification k₂ of the projection device and the first display interval D₁, and D₁=ΔL×tgα÷k₂.

As an optional embodiment, the fringe-encoded image further includes a second fringe group adjacent to the first fringe group. An imaging plane includes a second imaging interval adjacent to the first imaging interval. When the to-be-scanned object is located within the effective depth-of-field range of the three-dimensional scanning device, an image of the second fringe group on the imaging plane is located within the second imaging interval, and only the second fringe group exists in the second imaging interval.

Optionally, the first fringe group is projected to the near point of the depth of field of the projection device, and the second fringe group is projected to the far point of the depth of field of the projection device; or, the first fringe group is projected to the far point of the depth of field of the projection device, and the second fringe group is projected to the near point of the depth of field of the projection device.

As an optional embodiment, the fringe-encoded image includes a plurality of fringe groups periodically arranged, and first fringe groups and second fringe groups are respectively located in a cycle.

Optionally, the fringe-encoded image includes a time-encoded image or color-encoded image. The time-encoded image includes a plurality of time fringe patterns arranged based on time, and the color-encoded image includes a color fringe pattern encoded by a plurality of colors.

As an optional embodiment, binary encoding is adopted in the fringe-encoded image. In the time fringe image, pixels with fringes are denoted by a code 1, and pixels without fringes are denoted by a code 0. In the color-encoded pattern, pixels with red fringes (R) are denoted by a code 100, pixels with blue fringes (B) are denoted by a code 001, pixels with green fringes (G) are denoted by a code 010, and pixels without fringes are denoted by a code 000. Of course, if there are only two-color fringes, two-bit encoding may be adopted. For example, pixels with red fringes are denoted by a code 10, pixels with blue fringes are denoted by a code 01, and pixels without fringes are denoted by a code 00.

Optionally, when the fringe-encoded image is the time-encoded image, the time-encoded image includes the plurality of time fringe patterns which are sequentially projected according to a time sequence, wherein the plurality of time fringe patterns correspond to one encoding cycle.

Optionally, the first fringe group may be the time fringe pattern projected at the first time, and the second fringe group may be the time fringe pattern projected at the second time.

Optionally, the first fringe group may also be the color-encoded pattern.

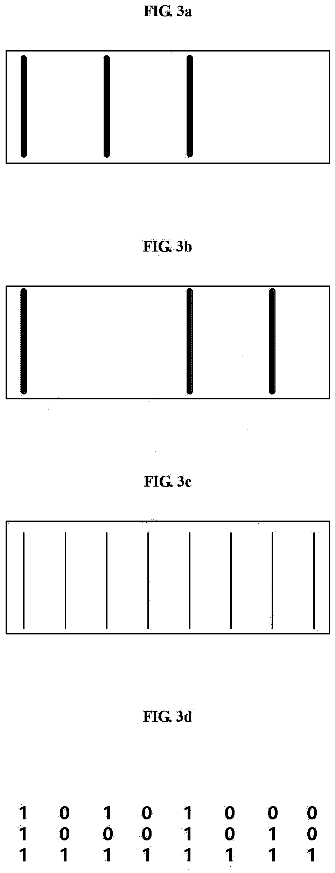

FIG. 3a is a schematic diagram I of a first time fringe pattern according to an embodiment of the present disclosure. FIG. 3b is a schematic diagram II of a second time fringe pattern according to an embodiment of the present disclosure. FIG. 3c is a schematic diagram III of a third time fringe pattern according to an embodiment of the present disclosure. As shown in FIG. 3a, FIG. 3b and FIG. 3c, the three time fringe patterns shown in FIG. 3a to FIG. 3c correspond to one encoding cycle, each fringe in the three time fringe patterns in the encoding cycle are decoded to obtain a time image encoding table, and the sequence of projected fringes can be determined according to the encoding table.

FIG. 3d is a schematic diagram of an encoding table of a time-encoded image according to an embodiment of the present disclosure. As shown in FIG. 3d, a binary fringe code shown in FIG. 3d is obtained by sequentially acquiring values (adopting binary encoding 0 or 1) at same pixel positions in the time fringe patterns shown in FIG. 3a to FIG. 3c, and arranging the three time fringe patterns according to an acquisition time sequence.

A single-cycle fringe code of the first time fringe pattern is 10101000, and 10101000 may be periodically and repeatedly set in the first time fringe pattern. A single-cycle fringe code of the second time fringe pattern is 10001010, and 10001010 may be periodically and repeatedly set in the second time fringe pattern. A single-cycle fringe code of the third time fringe pattern is 11111111, and 11111111 may be periodically and repeatedly set in the third time fringe pattern. Of course, 10101000, 10001010 and 11111111 are correspondingly the same in repeated cycle number. In a projection process, the three time fringe patterns are projected according to the time sequence, for example, the first time fringe pattern is projected at a first projection time, the second time fringe pattern is projected at a second projection time, and the third time fringe pattern is projected at a third projection time.

Optionally, when the fringe-encoded image of the camera image is obtained before three-dimensional reconstruction, fringe damages caused by object boundaries, occlusions, reflection and other various severe environments all can be recognized by above encoding, such that the problem about ambiguous encoding is solved.

It needs to be explained that the three time fringe-encoded images shown in FIG. 3a to FIG. 3c are designed into a reconstruction cycle, and decoding and reconstruction work can be finished based on the three time fringe-encoded images, thereby greatly shortening the time for continuously collecting the time fringe-encoded images during dynamic scanning, and solving the problems of image misalignment, image blurring, decoding errors, etc. caused by rapid movement.

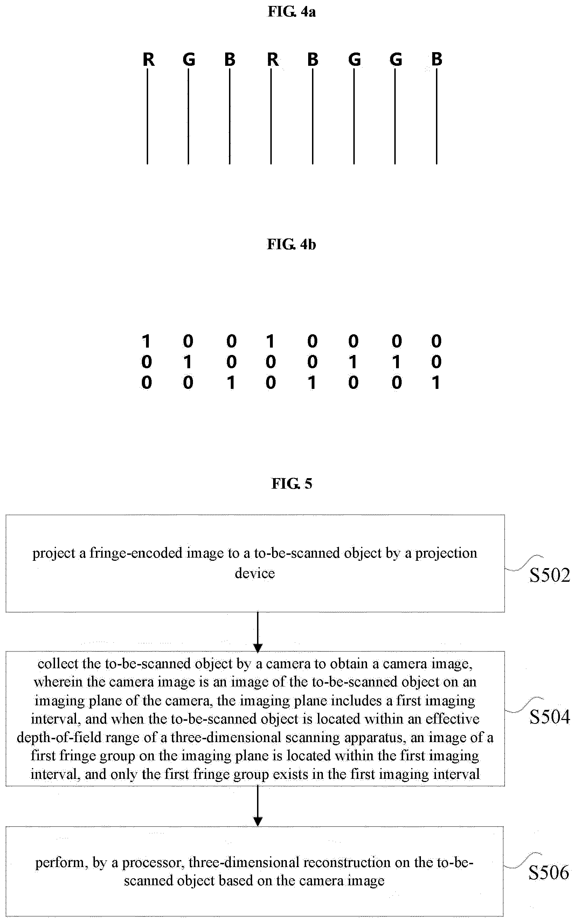

FIG. 4a is a schematic diagram of a color-encoded image according to an embodiment of the present disclosure. As shown in FIG. 4a, each fringe in an encoding cycle is subject to color encoding, and the greater the kinds of colors used, the easier it is to design unique encoding. However, this also increases the difficulty of color encoding recognition, as it is more difficult to distinguish the differences between colors when there are more color kinds. The number of the fringes is controlled, such as 8, that is, encoding distinguishing may be performed through three colors, thereby greatly reducing encoding and decoding complexity.

FIG. 4b is a schematic diagram of an encoding table of a color-encoded image according to an embodiment of the present disclosure. As shown in FIG. 4b, based on encoded values of different-colored fringe-encoded images (adopting binary encoding 0 or 1 to represent color three-channel information), a three-bit binary number is obtained, which is a fringe code.

For example, the fringe-encoded image shown in FIG. 4a has three colors, and each-color fringe corresponds to one encoding sequence, wherein an encoding sequence corresponding to a red fringe (R) is 100, an encoding sequence corresponding to a blue fringe (B) is 001, and an encoding sequence corresponding to a green fringe (G) is 010. Of course, the fringe-encoded image may also be a color fringe sequence arranged based on a DeBruijn sequence, or a plurality of fringe sequences repeatedly arranged with the color fringe sequence arranged based on the DeBruijn sequence as a single cycle.

Optionally, when the fringe-encoded image of the camera image is obtained before three-dimensional reconstruction, fringe damages caused by object boundaries, occlusions, reflection and other various severe environments all can be recognized by above encoding, such that the problem about ambiguous encoding is solved.

It needs to be explained that one simple color fringe-encoded image based on color encoding can be realized with the different colors of fringe-encoded images shown in FIG. 4a as one cycle, such that decoding and reconstruction can be implemented, the duration for collecting image sequences required for single-frame three-dimensional data during dynamic scanning is greatly shortened, encoding and decoding complexity and calculation losses are reduced, and the problems that due to many color kinds, an algorithm is complex and time-consuming, and decoding is in error are solved.

As an optional embodiment, a system included angle α is formed between a projection optical axis of a projection device and a collection optical axis of a camera. Optical parameters of the camera include lens magnification k₁, an effective depth of field ΔL of the three-dimensional scanning device and a second imaging interval d₂, and d₂=ΔL×tgα÷k₁.

As an optional embodiment, the projection device includes an image display element. The image display element includes a second display interval provided with a second fringe group. Optical parameters of the projection device include lens magnification k₂ of the projection device and the second display interval D₂, and D₂=ΔL×tgα÷k₂.

Optionally, the system included angle α ranges from 6 degrees to 10 degrees.

As an optional embodiment, a processor is configured to, based on the camera image, perform three-dimensional reconstruction on the to-be-scanned object.

As an optional embodiment, first imaging interval coordinates are preset in the processor. The processor determines, based on the camera image, pixel coordinates of a center of fringe in the camera image. The processor determines, based on the pixel coordinates of the fringes and the first imaging interval coordinates, a number of each fringe in the camera image. The processor performs, based on the pixel coordinates of the center of fringe and the number of each fringe, three-dimensional reconstruction to obtain a three-dimensional digital model of the to-be-scanned object.

As an optional embodiment, second imaging interval coordinates are preset in the processor. The processor determines, based on the camera image, the pixel coordinates of the center of fringe in the camera image. The processor determines, based on the pixel coordinates of the fringes, the first imaging interval coordinates and the second imaging interval coordinates, the number of each fringe in the camera image. The processor performs, based on the pixel coordinates and the number of the center of fringe , three-dimensional reconstruction to obtain a three-dimensional digital model of the to-be-scanned object.

As an optional embodiment, a light plane of each fringe and a corresponding number thereof in the fringe-encoded image are preset in the processor. The processor determines, based on the number of each fringe in the camera image and the corresponding number of the light plane of each fringe, a light plane corresponding to the pixel coordinates of the center of each fringe. The processor performs, based on the pixel coordinates of the center of each fringe and the corresponding light plane, trigonometric calculation to reconstruct a three-dimensional digital model of the to-be-scanned object.

FIG. 5 is a flowchart I of a three-dimensional scanning method according to an embodiment of the present disclosure. As shown in FIG. 5, the method performs, based on the above three-dimensional scanning device, following steps:
Step S502: A fringe-encoded image is projected to a to-be-scanned object by a projection device.
Step S504: The to-be-scanned object is collected by a camera to obtain a camera image, wherein the camera image is an image of the to-be-scanned object on an imaging plane of the camera, the imaging plane includes a first imaging interval, and when the to-be-scanned object is located within an effective depth-of-field range of a three-dimensional scanning device, an image of a first fringe group on the imaging plane is located within the first imaging interval, and only the first fringe group exists in the first imaging interval.
Step S506: A processor performs, based on the camera image, three-dimensional reconstruction on the to-be-scanned object.

In this embodiment of the present disclosure, the projection device is configured to project the fringe-encoded image to the to-be-scanned object, wherein the fringe-encoded image includes the first fringe group. The camera is configured to collect the to-be-scanned object to obtain the camera image, wherein the camera image is the image of the to-be-scanned object on the imaging plane of the camera, and the imaging plane includes the first imaging interval. When the to-be-scanned object is located within the effective depth-of-field range of the three-dimensional scanning device, the image of the first fringe group on the imaging plane is located within the first imaging interval, and only the first fringe group exists in the first imaging interval. The three-dimensional scanning device restricts, according to the linear propagation characteristic of light, the fringe-encoded image within the first imaging interval defined by a hardware structure of the three-dimensional scanning device. Thus, by utilizing the first imaging interval as an encoding cycle and ensuring unique encoding of the fringe-encoded image in the encoding cycle, the unique encoding can be guaranteed by utilizing a small amount of encoding information (i.e., fewer sequence images or less space codes) of the fringe-encoded image. Accordingly, the three-dimensional scanning device can be used by combining optical characteristics without relying on a high-difficulty hardware level, and dynamic scanning speed may also be increased by fewer image sequences or a simple space encoding and decoding method, thereby realizing a technical effect of improving the scanning efficiency, and then solving the technical problem that in the related art, the multiple image sequences are required to be complexly encoded to generate the structured light encoding pattern. As an optional embodiment, the method further includes: pixel coordinates of a center of each fringe in a camera image are determined based on the camera image; first imaging interval coordinates are preset in the processor, and a number of each fringe are determined based on the pixel coordinates of the fringes and the first imaging interval coordinates; and three-dimensional reconstruction is performed on the pixel coordinates of the center of each fringe based on the numbers to obtain a three-dimensional digital model of the to-be-scanned object.

As an optional embodiment, the camera collects the to-be-scanned object to obtain a camera image, wherein the camera image is an image of the to-be-scanned object on an imaging plane of the camera, and the imaging plane includes a first imaging interval and a second imaging interval. When the to-be-scanned object is located within an effective depth-of-field range of the three-dimensional scanning device, an image of a first fringe group on the imaging plane is located within the first imaging interval, and only the first fringe group exists in the first imaging interval; and an image of a second fringe group on the imaging plane is located within the second imaging interval, and only the second fringe group exists in the second imaging interval. It needs to be explained that when the to-be-scanned object moves within the effective depth of field range, the first fringe group moves within the first imaging interval but does not exceed the first imaging interval all the time, and the second fringe group moves within the second imaging interval but does not exceed the second imaging interval all the time.

As an optional embodiment, a projection device is configured to project a fringe-encoded image to a to-be-scanned object, wherein the fringe-encoded image includes a first fringe group and a second fringe group. A camera is configured to collect the to-be-scanned object to obtain a camera image, wherein the camera image is an image of the to-be-scanned object on an imaging plane of the camera, and the imaging plane includes a first imaging interval and a second imaging interval. When the to-be-scanned object is located within an effective depth-of-field range of the three-dimensional scanning device, an image of the first fringe group on the imaging plane is located within the first imaging interval, and only the first fringe group exists in the first imaging interval; and an image of the second fringe group on the imaging plane is located within the second imaging interval, and only the second fringe group exists in the second imaging interval. The three-dimensional scanning device restricts, according to the linear propagation characteristic of light, the fringe-encoded image within the first imaging interval and the second imaging interval defined by a hardware structure of the three-dimensional scanning device. Thus, by utilizing the first imaging interval for imaging only in one encoding cycle, utilizing the second imaging interval for imaging only in another encoding cycle and ensuring unique encoding of the fringe-encoded image in each encoding cycle, the unique encoding can be guaranteed by utilizing a small amount of encoding information (i.e., fewer sequence images or less space codes) of the fringe-encoded image. Accordingly, the three-dimensional scanning device can be used by combining optical characteristics without relying on a high-difficulty hardware level, and dynamic scanning speed may also be increased by fewer image sequences or a simple space encoding and decoding method, thereby realizing a technical effect of improving the scanning efficiency, and then solving the technical problem that in the related art, the multiple image sequences are required to be complexly encoded to generate the structured light encoding pattern. Sequence fringes with unique encoding may be repeatedly set in a same projection pattern, such that encoding difficulty is reduced.

As an optional embodiment, the method further includes: pixel coordinates of a center of each fringe in a camera image are determined based on the camera image; coordinates of a first imaging interval and a second imaging interval are preset in the processor, and a number of each fringe are determined based on the pixel coordinates of the fringes and the coordinates of the first imaging interval and the second imaging interval; and three-dimensional reconstruction is performed on the pixel coordinates of the center of each fringe based on the numbers to obtain a three-dimensional digital model of the to-be-scanned object.

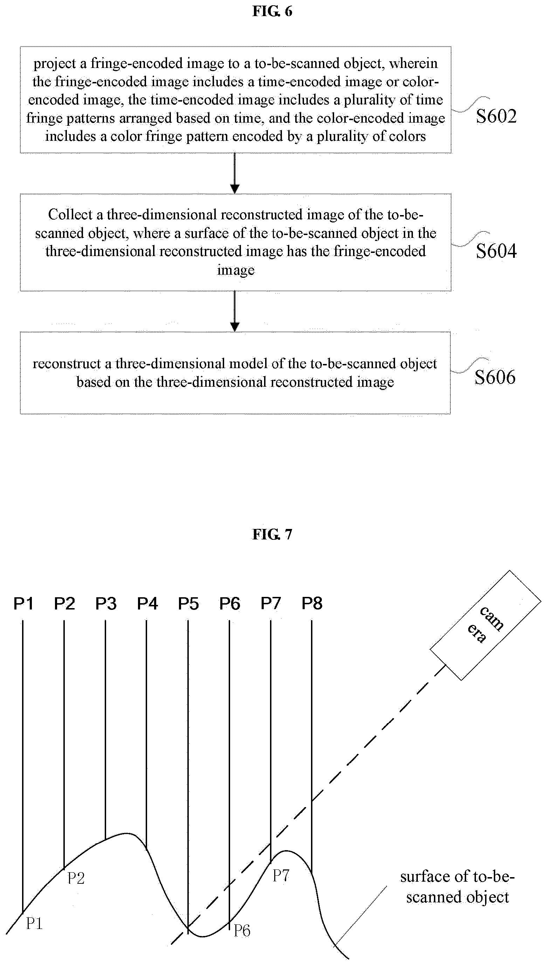

FIG. 6 is a flowchart II of a three-dimensional scanning method according to an embodiment of the present disclosure. As shown in FIG. 6, the method includes following steps:
Step S602: A fringe-encoded image is projected to a to-be-scanned object, wherein the fringe-encoded image includes a time-encoded image or color-encoded image, the time-encoded image includes a plurality of time fringe patterns arranged based on time, and the color-encoded image includes a color fringe pattern encoded by a plurality of colors.
Step S604: A three-dimensional reconstructed image of the to-be-scanned object is collected, wherein a surface of the to-be-scanned object in the three-dimensional reconstructed image has the fringe-encoded image.
Step S606: A three-dimensional model of the to-be-scanned object is reconstructed based on the three-dimensional reconstructed image.

The fringe-encoded image is projected to the to-be-scanned object and is modulated by the to-be-scanned object and deformed, the obtained three-dimensional reconstructed image of the to-be-scanned object is a surface image of the scanned object, and the image includes the deformed fringe-encoded image.

In this embodiment of the present disclosure, the fringe-encoded image is projected to the to-be-scanned object and includes the time-encoded image or color-encoded image, the time-encoded image includes the plurality of time fringe patterns arranged based on time, and the color-encoded image includes a color fringe pattern encoded by a plurality of colors. The three-dimensional reconstructed image of the to-be-scanned object is collected, wherein the surface of the to-be-scanned object in the three-dimensional reconstructed image has the fringe-encoded image. The three-dimensional model of the to-be-scanned object is reconstructed based on the three-dimensional reconstructed image, such that through the time-encoded image or color-encoded image, the fringe-encoded image can have a unique fringe code, thereby achieving the purpose of ensuring the unique fringe encoding of the fringe-encoded image, realizing the technical effect of increasing dynamic scanning speed, and then solving the technical problem that encoding of required projection images in the three-dimensional scanning process is complex.

Optionally, in the color-encoded image, the color fringe pattern at least includes a first fringe group.

Optionally, in the time-encoded image, a time fringe pattern projected at the first time may be the first fringe group, and a time fringe pattern projected at the second time may be a second fringe group.

FIG. 7 is a schematic diagram of encoding occlusions according to an embodiment of the present disclosure. As shown in FIG. 7, P1-P8 denote encoding fringes, wherein due to an object (i.e., a to-be-scanned object) obstructing the camera view at P1 and P2, there is a phenomenon of broken edges in the edge fringes, resulting in incompleteness of single-frame data. In addition, encoding information at P1 to P2 is very close to encoding information at P6 to P7, resulting in ambiguous encoding, and noise and cluttered data during three-dimensional reconstruction. But based on the technical solution provided by the present application, the fringe code can be recognized based on an image encoding table (e.g., a time image encoding table or a color image encoding table), thereby improving the efficiency of fringe code recognition.

In the above step S604, collecting the three-dimensional reconstructed image of the to-be-scanned object includes collecting one or more images obtained after projecting the fringe-encoded image to the to-be-scanned object, wherein when the fringe-encoded image is the time-encoded image, a plurality of images with surfaces having the fringe-encoded image may be collected, and the three-dimensional reconstructed image is determined based on the plurality of collected images; and when the fringe-encoded image is the color-encoded image, one image with a surface having the fringe-encoded image may be collected, and the three-dimensional reconstructed image is determined based on the image.

The above step S606 that a three-dimensional model of the to-be-scanned object is reconstructed based on the three-dimensional reconstructed image includes: adopting a monocular stereoscopic vision reconstruction system or a binocular stereoscopic vision system to reconstruct the three-dimensional model.

For example, in the process of reconstructing the three-dimensional model based on the binocular stereoscopic vision system, the binocular stereoscopic vision system includes a camera A and a camera B. In the process of collecting the three-dimensional reconstructed image of the to-be-scanned object, a three-dimensional reconstructed image collected by the camera A is a first three-dimensional reconstructed image, a three-dimensional reconstructed image collected by the camera B is a second three-dimensional reconstructed image, and then the three-dimensional model of the to-be-scanned object is reconstructed based on common fringe codes in the first three-dimensional reconstructed image and the second three-dimensional reconstructed image.

For another example, in the process of reconstructing the three-dimensional model based on the monocular stereoscopic vision system, the camera collects the three-dimensional reconstructed image, and the three-dimensional model of the to-be-scanned object is reconstructed based on fringes and corresponding light plane in the three-dimensional reconstructed image.

To facilitate illustration in the following description, content projected to the surface of the to-be-scanned object serves as fringe patterns wherein the fringe patterns include time fringe patterns (e.g., a first time fringe pattern, a second time fringe pattern, a third time fringe pattern, a fourth time fringe pattern and a fifth time fringe pattern) and color fringe patterns (e.g., a first color fringe pattern and a second color fringe pattern). Collected content with the to-be-scanned object serves as fringe images, wherein the fringe images have the to-be-scanned object, the surface of the to-be-scanned object has fringe patterns, and the fringe images include time fringe images (e.g., a first time fringe image, a second time fringe image, a third time fringe image, a fourth time fringe image and a fifth time fringe image) and color fringe images (e.g., a first color fringe image and a second color fringe image).

For example, after the first time fringe pattern is projected to the to-be-scanned object, the surface of the to-be-scanned object has the projected first time fringe pattern, and at the time, the image of the to-be-scanned object (i.e., the first time fringe image) is collected, such that the collected first time fringe image has the to-be-scanned object and the first time fringe pattern projected to the surface of the to-be-scanned object.

The relationship between other fringe patterns and fringe images is similar to the above relationship, which is not described in detail herein.

As an optional embodiment, when the fringe-encoded image is the time-encoded image, the three-dimensional scanning method further includes: the first time fringe pattern is projected to the surface of the to-be-scanned object at the first time; the first time fringe image on the surface of the to-be-scanned object is obtained; the second time fringe pattern is projected to the surface of the to-be-scanned object at the second time; the second time fringe image on the surface of the to-be-scanned object is obtained; and a time image encoding table is determined based on the first time fringe image and the second time fringe image.

Optionally, the first time is earlier than the second time.

According to the above embodiment of the present disclosure, the first time fringe pattern is projected to the surface of the to-be-scanned object at the first time, and the first time fringe image on the surface of the to-be-scanned object is obtained; the second time fringe pattern is projected to the surface of the to-be-scanned object at the second time, and the second time fringe image on the surface of the to-be-scanned object is obtained, such that the image encoding table is jointly defined based on the first time fringe image and the second time fringe image according to a time sequence.

It needs to be explained that the collected first time fringe image refers to the first three-dimensional reconstructed image, and the first three-dimensional reconstructed image includes the first time fringe pattern modulated by the to-be-scanned object; and the collected second time fringe image refers to the second three-dimensional reconstructed image, and the second three-dimensional reconstructed image includes the second time fringe pattern modulated by the to-be-scanned object.

As an optional embodiment, the operation of determining the time image encoding table based on the first time fringe image and the second time fringe image includes: a first encoding table is determined based on the first time fringe image; a second encoding table is determined based on the second time fringe image; and the time image encoding table is constructed based on the first encoding table and the second encoding table.

As an optional embodiment, the step that a first encoding table is determined based on the first time fringe image includes: first encoded values are correspondingly assigned to pixels with fringes in the first time fringe image, second encoded values are correspondingly assigned to pixels without fringes in the first time fringe image, and the first encoding table is constructed by the first encoded values and the second encoded values based on pixel position distribution of the first time fringe image. The step that a second encoding table is determined based on the second time fringe image includes: first encoded values are correspondingly assigned to pixels with fringes in the second time fringe image, second encoded values are correspondingly assigned to pixels without fringes in the second time fringe image, and the second encoding table is constructed by the first encoded values and the second encoded values based on the pixel position distribution of the second time fringe image. The step that the time image encoding table is constructed based on the first encoding table and the second encoding table includes: the encoded values at same pixel positions in the first encoding table and the second encoding table are arranged according to an obtaining sequence of the first time fringe image and the second time fringe image to serve as encoding sequences of corresponding pixels, and the time image encoding table is constructed based on the encoding sequences.

As an optional embodiment, the encoding table adopts binary encoding, encoded values corresponding to pixels with fringes in the time-encoded image are denoted by 1, and encoded values corresponding to pixels without fringes in the time-encoded image are denoted by 0.

According to the above embodiment of the present disclosure, a plurality of pixel positions are arranged in the time fringe patterns (e.g., the first time fringe pattern and the second time fringe pattern), and each pixel can represent binary encoding. For example, if the pixels with fringes are distributed at the pixel position, which are represented by a first encoded value, such as 1; and if the pixels without fringes are distributed at the pixel position, which are represented by a second encoded value, such as 0. Thus, the corresponding first encoding table is achieved based on the first time fringe image, and the corresponding second encoding table is achieved based on the second time fringe image. Accordingly, based on the first encoding table and the second encoding table, the corresponding encoding sequences of same pixel positions can be obtained according to the fringe obtaining sequence to constitute the time image encoding table.

For example, a pixel position A in the first time fringe image is encoded as 1, and a position B is encoded as 0; and a pixel position A in the second time fringe image is encoded as 0, and a position B is encoded as 1. Thus, the first encoding table corresponding to the first time fringe image is (A: 1, B:0), and the second encoding table corresponding to the second time fringe image is (A:0, B:1). Accordingly, the time image encoding table determined based on the first encoding table and the second encoding table is (A: 10, B:01).

Optionally, there may be two or more projected time fringe patterns, and the plurality of time fringe patterns are sequentially arranged according to the time sequence, thereby generating a multi-bit code.

As an optional embodiment, after the second time fringe image on the surface of the to-be-scanned object is obtained, the method further includes: the third time fringe pattern is projected to the surface of the to-be-scanned object at the third time; the third time fringe image on the surface of the to-be-scanned object is obtained; and a time image encoding table is determined based on the first time fringe image, the second time fringe image and the third time fringe image.

For example, the pixel position A in the first time fringe image is encoded as 1, and the position B is encoded as 0; the pixel position A in the second time fringe image is encoded as 0, and the position B is encoded as 1; and a pixel position A in the third time fringe image is encoded as 1, and a position B is encoded as 1. Thus, the first encoding table corresponding to the first time fringe image is (A:1, B:0), the second encoding table corresponding to the second time fringe image is (A:0, B: 1), and the third encoding table corresponding to the third time fringe image is (A:1, B:1). Accordingly, the image encoding table determined based on the first encoding table, the second encoding table and the third encoding table is (A:101, B:011).

As an optional embodiment, the step that a time image encoding table is determined based on the first time fringe image, the second time fringe image and the third time fringe image includes: first encoded values are correspondingly assigned to pixels with fringes in the first time fringe image, second encoded values are correspondingly assigned to pixels without fringes in the first time fringe image, and a first encoding table is constructed by the first encoded values and the second encoded values based on pixel position distribution of the first time fringe image; first encoded values are correspondingly assigned to pixels with fringes in the second time fringe image, second encoded values are correspondingly assigned to pixels without fringes in the second time fringe image, and a second encoding table is constructed by the first encoded values and the second encoded values based on pixel position distribution of the second time fringe image; first encoded values are correspondingly assigned to pixels with fringes in the third time fringe image, second encoded values are correspondingly assigned to pixels without fringes in the third time fringe image, and a third encoding table is constructed by the first encoded values and the second encoded values based on pixel position distribution of the third time fringe image; and the encoded values at same pixel positions in the first encoding table, the second encoding table and the third encoding table are arranged according to an obtaining sequence of the first time fringe image, the second time fringe image and the third time fringe image to serve as encoding sequences of corresponding pixels, and the time image encoding table is constructed based on the encoding sequences.

As an optional embodiment, after a time image encoding table is determined based on the first time fringe image and the second time fringe image, the method further includes: the fourth time fringe pattern is projected to the surface of the to-be-scanned object to obtain the fourth time fringe pattern on the surface of the to-be-scanned object, and a sequence of each fringe in the fourth time fringe image is determined based on the time image encoding table; and the fifth time fringe pattern is projected to the surface of the to-be-scanned object to obtain the fifth time fringe pattern on the surface of the to-be-scanned object, and a sequence of each fringe in the fifth time fringe image is determined based on the time image encoding table, wherein the fifth time fringe pattern is obtained by deflecting the fringes in the fourth time fringe pattern by a distance d in a same direction.

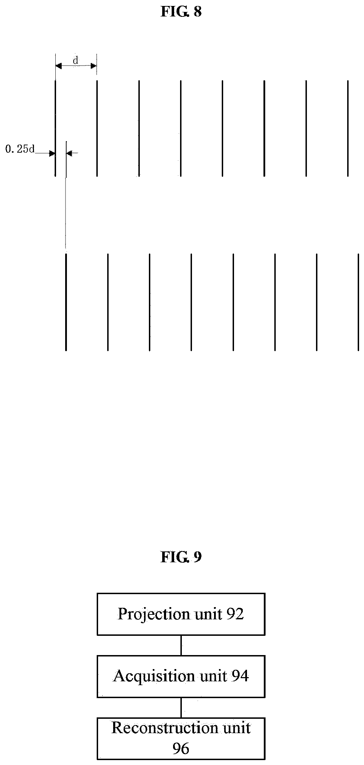

FIG. 8 is a schematic diagram of reconstructed fringe offset according to an embodiment of the present disclosure. As shown in FIG. 8, assuming that fringe spacing is L, reconstructed fringes may be designed into a dense fringe group with equidistant offset, such that the dense degree of single-frame data is increased. The offset distance d of the fringes may be designed as 1/2, 1/3, 1/4, etc. of L according to the requirement for the fringe resolution, and the smaller the fringe offset distance, the higher the resolution; and the larger the fringe offset distance, the smaller the number of fringe images, the higher the scanning speed.

Optionally, based on the three-dimensional scanning device shown in FIG. 1, device parameters such as the effective depth of field of a projection system, lens magnification of the camera and the optical included angle between the projection optical axis of the projection system and the camera shooting optical axis of the camera are decided by physical properties of hardware in the three-dimensional scanning device, and based on the above device parameters, the fringe-encoded image moves in an image plane of the camera.

Accordingly, the fringe-encoded image cannot exceed a collection range of the camera based on the device parameters of the above three-dimensional scanning device, thereby facilitating three-dimensional reconstruction on the collected image of the to-be-scanned object with the fringe code.

It needs to be explained that in a scanning scenario with a small range of field of view, a structured light time fringe pattern (i.e., the fringe-encoded image) with a same encoded value inevitably moves in the image plane of the camera within the effective range of depth of field due to the included angle of the binocular system and the magnification of the optical lens, and the movement range is decided by three aspects: the effective depth of field, the included angle of the optical system and the magnification of the lens. After the parameters of the optical system are fixed, the movement range (i.e., the offset distance) is determined, and by designing unique fringe encoding within the movement range (i.e., the offset distance), the unique encoded value across the entire image plane can be guaranteed. Due to the linear propagation characteristic of light, projection light rays within the movement range (i.e., the offset distance) cannot jump out of the range. The movement range (i.e., the offset distance) is utilized as one encoding cycle, unique encoding is guaranteed in the encoding cycle, and because the encoding cycle can be ensured to be short according to optical design, the unique encoding can be guaranteed by utilizing a small amount of encoding information (fewer sequence images or less space codes). Because fringes in other encoding cycles cannot interfere with fringes in this encoding cycle within a global range, a plurality of encoding cycles are usually adopted in the entire image plane.

As an optional embodiment, when the fringe-encoded image is the color-encoded image, the three-dimensional scanning method includes: the color-encoded image is projected to the surface of the to-be-scanned object, wherein the color-encoded image includes the first color fringe pattern and the second color fringe pattern; the color fringe images on the surface of the to-be-scanned object are obtained, wherein the color fringe images include the first color fringe image and the second color fringe image; and a color image encoding table is determined based on the first color fringe image and the second color fringe image.

It needs to be explained that the first color fringe image and the second color fringe image are formed by acquiring, through corresponding color channels, multiple colors of fringes in a same color fringe pattern. For example, one color fringe pattern includes a combined arrangement of red fringes and green fringes, a red channel of the camera obtains the red fringes to form a red fringe image, and a green channel of the camera obtains the green fringes to form a green fringe image.

As an optional embodiment, the step that a color image encoding table is determined based on color fringe images includes: a first color encoding table is determined based on the first color fringe image; a second color encoding table is determined based on the second color fringe image; and the color image encoding table is constructed based on the first color encoding table and the second color encoding table.

As an optional embodiment, the step that a first color encoding table is determined based on the first color fringe image includes a first encoding sequence is correspondingly assigned to pixels with a first color in the first color fringe image, a fourth encoding sequence is correspondingly assigned to pixels without the first color in the first color fringe image, and the first color encoding table is constructed by the first encoding sequence and the fourth encoding sequence based on pixel position distribution of the first color fringe image. The step that a second color encoding table is determined based on the second color fringe image includes: a second encoding sequence is correspondingly assigned to pixels with a second color in the second color fringe image, a fourth encoding sequence is correspondingly assigned to pixels without the second color in the second color fringe image, and the second color encoding table is constructed by the second encoding sequence and the fourth encoding sequence based on pixel position distribution of the second color fringe image. The step that the color image encoding table is constructed based on the first color encoding table and the second color encoding table includes: superposing the encoding sequence at same pixel positions in the first color encoding table and the second color encoding table as encoding sequences of corresponding pixels, and constituting the color image encoding table based on superimposed encoding sequences corresponding to a distribution of each pixel.

As an optional embodiment, the encoding table adopts binary encoding. The first encoding sequence corresponding to the pixels with the first color in the color-encoded image is (0, 0, 1), the second encoding sequence corresponding to the pixels with the second color in the color-encoded image is (0, 1, 0), and the fourth encoding sequence corresponding to the pixels without colors in the color-encoded image is (0, 0, 0).

According to another aspect of this embodiment of the present disclosure, a computer-readable storage medium is further provided and includes stored programs. The programs, when running, control a device where the computer-readable storage medium is located to execute the above three-dimensional scanning method.

According to another aspect of this embodiment of the present disclosure, a processor is further provided. The processor is configured to run programs. The programs, when running, execute the above three-dimensional scanning method.

According to this embodiment of the present disclosure, an embodiment of a three-dimensional scanning apparatus is further provided. It needs to be explained that the three-dimensional scanning apparatus may be configured to execute the three-dimensional scanning method in this embodiment of the present disclosure, and the three-dimensional scanning method in this embodiment of the present disclosure may be executed in the three-dimensional scanning apparatus.

FIG. 9 is a schematic diagram of a three-dimensional scanning apparatus according to an embodiment of the present disclosure. As shown in FIG. 9, the apparatus may include:
a projection unit 92, configured to project a fringe-encoded image to a to-be-scanned object, wherein the fringe-encoded image includes a time-encoded image or color-encoded image, the time-encoded image includes a plurality of time fringe patterns arranged based on time, and the color-encoded image includes a color fringe pattern encoded by a plurality of colors; an acquisition unit 94, configured to collect a three-dimensional reconstructed image of the to-be-scanned object, wherein a surface of the to-be-scanned object in the three-dimensional reconstructed image has the fringe-encoded image; and a reconstruction unit 96, configured to reconstruct, based on the three-dimensional reconstructed image, a three-dimensional model of the to-be-scanned object.

It needs to be explained that the projection unit 92 in this embodiment may be configured to perform step S602 in this embodiment of the present application, the acquisition unit 94 in this embodiment may be configured to perform step S604 in this embodiment of the present application, and the reconstruction unit 96 in this embodiment may be configured to perform step S606 in this embodiment of the present application. Examples and application scenarios implemented by the above device and corresponding steps are the same, but are not limited to the content disclosed by the above embodiments.

In this embodiment of the present disclosure, the fringe-encoded image is projected to the to-be-scanned object and includes the time-encoded image or color-encoded image. The time-encoded image includes the plurality of time fringe patterns arranged based on time, and the color-encoded image includes a color fringe pattern encoded by a plurality of colors. The three-dimensional reconstructed image of the to-be-scanned object is collected, wherein the surface of the to-be-scanned object in the three-dimensional reconstructed image has the fringe-encoded image. The three-dimensional model of the to-be-scanned object is reconstructed based on the three-dimensional reconstructed image, such that through the time-encoded image and color-encoded image, the fringe-encoded image can have a unique fringe code, thereby achieving the purpose of ensuring unique fringe encoding of the fringe-encoded image, realizing the technical effect of increasing dynamic scanning speed, and then solving the technical problem that encoding of required projection images in the three-dimensional scanning process is complex.

As an optional embodiment, when the fringe-encoded image is the time-encoded image, the three-dimensional scanning apparatus further includes: a first projection unit, configured to project a first time fringe pattern to the surface of the to-be-scanned object at the first time; a first acquiring unit, configured to obtain a first time fringe image on the surface of the to-be-scanned object; a second projection unit, configured to project a second time fringe pattern to the surface of the to-be-scanned object at the second time; a second acquiring unit, configured to obtain a second time fringe image on the surface of the to-be-scanned object; and a first determining unit, configured to determine a time image encoding table based on the first time fringe image and the second time fringe image.

As an optional embodiment, the first determining unit includes: a first determining module, configured to determine a first encoding table based on the first time fringe image; a second determining module, configured to determine a second encoding table based on the second time fringe image; and a first construction module, configured to construct a time image encoding table based on the first encoding table and the second encoding table.

As an optional embodiment, the first determining module includes: a first determining submodule, configured to correspondingly assign first encoded values to pixels with fringes in the first time fringe image, correspondingly assign second encoded values to pixels without fringes in the first time fringe image, and construct the first encoding table by the first encoded values and the second encoded values based on pixel position distribution of the first time fringe image. The second determining module includes: a second determining submodule, configured to correspondingly assign first encoded values to pixels with fringes in the second time fringe image, correspondingly assign second encoded values to pixels without fringes in the second time fringe image, and construct the second encoding table by the first encoded values and the second encoded values based on pixel position distribution of the second time fringe image. The first construction module includes: a first construction submodule, configured to arrange the encoded values at same pixel positions in the first encoding table and the second encoding table according to an obtaining sequence of the first time fringe image and the second time fringe image to serve as encoding sequences of corresponding pixels, and construct the time image encoding table based on the encoding sequences.

As an optional embodiment, the apparatus further includes: a third projection unit, configured to project a third time fringe pattern to the surface of the to-be-scanned object at the third time after the second time fringe image on the surface of the to-be-scanned object is obtained; a third acquiring unit, configured to obtain a third time fringe image on the surface of the to-be-scanned object; and a second determining unit, configured to determine a time image encoding table based on the first time fringe image, the second time fringe image and the third time fringe image.

As an optional embodiment, the second determining unit includes: a first encoding module, configured to correspondingly assign first encoded values to pixels with fringes in the first time fringe image, correspondingly assign second encoded values to pixels without fringes in the first time fringe image, and construct the first encoding table by the first encoded values and the second encoded values based on pixel position distribution of the first time fringe image; a second encoding module, configured to correspondingly assign first encoded values to pixels with fringes in the second time fringe image, correspondingly assign second encoded values to pixels without fringes in the second time fringe image, and construct the second encoding table by the first encoded values and the second encoded values based on pixel position distribution of the second time fringe image; a third encoding module, configured to correspondingly assign first encoded values to pixels with fringes in a third time fringe image, correspondingly assign second encoded values to pixels without fringes in the third time fringe image, and construct a third encoding table by the first encoded values and the second encoded values based on pixel position distribution of the third time fringe image; and a fourth encoding module, configured to arrange the encoded values at same pixel positions in the first encoding table, the second encoding table and the third encoding table according to an obtaining sequence of the first time fringe image, the second time fringe image and the third time fringe image to serve as encoding sequences of corresponding pixels, and construct a time image encoding table based on the encoding sequences.

As an optional embodiment, the encoding table adopts binary encoding. In the time-encoded image, encoded values corresponding to pixels with fringes are denoted by 1, and encoded values corresponding to pixels without fringes are denoted by 0.

As an optional embodiment, the apparatus further includes: a third determining unit, configured to project a fourth time fringe pattern to the surface of the to-be-scanned object to obtain a fourth time fringe image on the surface of the to-be-scanned object after the time image encoding table is determined based on the first time fringe image and the second time fringe image, and determine a sequence of each fringe in the fourth time fringe image based on the time image encoding table; and a fourth determining unit, configured to project a fifth time fringe pattern to the surface of the to-be-scanned object to obtain a fifth time fringe image on the surface of the to-be-scanned object, and determine a sequence of each fringe in the fifth time fringe image based on the time image encoding table, wherein the fifth time fringe pattern is obtained by deflecting the fringes in the fourth time fringe pattern by a distance d in a same direction.

As an optional embodiment, when the fringe-encoded image is the color-encoded image, the three-dimensional scanning apparatus further includes: a fourth projection unit, configured to project the color-encoded image to the surface of the to-be-scanned object, wherein the color-encoded image includes a first color fringe pattern and a second color fringe pattern; a fourth acquiring unit, configured to obtain a color fringe image on the surface of the to-be-scanned object, wherein the color-encoded image includes a first color fringe image and a second color fringe image; and a fifth determining unit, configured to determine a color image encoding table based on the first color fringe image and the second color fringe image.

As an optional embodiment, the fifth determining unit includes: a third determining module, configured to determine a first color encoding table based on the first color fringe image; a fourth determining module, configured to determine a second color encoding table based on the second color fringe image; and a second construction module, configured to construct a color image encoding table based on the first color encoding table and the second color encoding table.

As an optional embodiment, the third determining module includes: a third determining submodule, configured to correspondingly assign a first encoding sequence to pixels with a first color in the first color fringe image, correspondingly assign a fourth encoding sequence to pixels without the first color in the first color fringe image, and construct a first color encoding table by the first encoding sequence and the fourth encoding sequence based on pixel position distribution of the first color fringe image. The fourth determining module includes: a fourth determining submodule, configured to correspondingly assign a second encoding sequence to pixels with a second color in the second color fringe image, correspondingly assign a fourth encoding sequence to pixels without the second color in the second color fringe image, and construct a second color encoding table by the second encoding sequence and the fourth encoding sequence based on pixel position distribution of the second color fringe image. The second construction module includes: a second construction submodule, configured to superpose the encoding sequence at same pixel positions in the first color encoding table and the second color encoding table as encoding sequences of corresponding pixels, and constituting the color image encoding table based on superimposed encoding sequences corresponding to a distribution of each pixel.

As an optional embodiment, the encoding table adopts binary encoding. The first encoding sequence corresponding to the pixels with the first color in the color-encoded image is (0, 0, 1), the second encoding sequence corresponding to the pixels with the second color in the color-encoded image is (0, 1, 0), and the fourth encoding sequence corresponding to the pixels without colors in the color-encoded image is (0, 0, 0).

As an optional embodiment, the first imaging intervals and the second imaging intervals are arranged at equal intervals.

The serial numbers of the above embodiments of the present disclosure are merely used for descriptions instead of representing good or bad of the embodiments.

In the above embodiments of the present disclosure, special emphasis is laid on a description of each embodiment, and for parts not described in detail in one embodiment, please refer to related descriptions in other embodiments.

It is to be understood that technical contents disclosed by the several embodiments provided by the present application may be implemented by other manners. The above described embodiments of an apparatus are merely schematic, such as unit division which may be logic function division; and during practical implementation, there may be additional division manners, for example, a plurality of units or components may be combined or integrated into another system, or some characteristics may be ignored or not executed. In addition, shown or discussed mutual coupling or direct coupling or communication connection may be realized through some interfaces, and unit or module indirect coupling or communication connection may be in an electrical form or other forms.

Units described as separation parts may be or may be not physically separated, and parts for unit display may be or may be not physical units, may be located at the same position, or may be distributed on a plurality of units. Part or all of the units may be selected according to actual demands to achieve objectives of the schemes of the embodiments.

In addition, functional units in the embodiments of the present disclosure may be integrated in one processing unit, or independently and physically exist, or two or more units may be integrated in one unit. The above integrated unit may be realized in a hardware form or a form of a software functional unit.

When the integrated unit is realized in the form of the software functional unit and serves as an independent product to be sold or used, the integrated unit may be stored in the computer-readable storage medium. Based on the understanding, the technical schemes of the present disclosure essentially or parts making contribution to the related art or all or part of the technical schemes may be embodied in a software product form. A computer software product is stored in a storage medium and includes a plurality of instructions for making a computer device (a personal computer, a server, or a network device, or the like) perform all or part of the steps of the methods in the embodiments of the present disclosure. The foregoing storage medium includes a U disk, a Read-Only Memory (ROM), a Random Access Memory (RAM), a mobile hard disk, a diskette or a light disk or other media capable of storing program code.

The above contents are merely preferred implementations of the present disclosure. It needs to be indicated that a plurality of improvements and embellishments may be made by those of ordinary skill in the art without departing from the principle of the present disclosure and should fall within the scope of protection of the present disclosure.

### Industrial applicability

The solutions provided by the embodiments of the present disclosure may be applied to the three-dimensional scanning process. The embodiments of the present disclosure solve the technical problem that in the related art, multiple image sequences are required to be complexly encoded to generate a structured light encoding pattern, and effectively improve the scanning efficiency.

## Claims

1. A three-dimensional scanning device, comprising:
a projection device, configured to project a fringe-encoded image to a to-be-scanned object, wherein the fringe-encoded image comprises a first fringe group; and
a camera, configured to collect the to-be-scanned object to obtain a camera image, wherein the camera image is an image of the to-be-scanned object on an imaging plane of the camera, the imaging plane comprises a first imaging interval, and in a case that the to-be-scanned object is located within an effective depth-of-field range of the three-dimensional scanning device, an image of the first fringe group on the imaging plane is located within the first imaging interval, and only the first fringe group exists in the first imaging interval.

2. The three-dimensional scanning device as claimed in claim 1, wherein
a system included angle α is formed between a projection optical axis of the projection device and a collection optical axis of the camera, optical parameters of the camera comprise lens magnification k₁, an effective depth of field ΔL of the three-dimensional scanning device and the first imaging interval di, and d₁=ΔL×tgα÷k₁.

3. The three-dimensional scanning device as claimed in claim 1, wherein
the projection device comprises an image display element, the image display element comprising a first display interval provided with the first fringe group; and
optical parameters of the projection device comprise lens magnification k₂ of the projection device and the first display interval D₁, and D₁=ΔL_{X}tgα÷k₂.

4. The three-dimensional scanning device as claimed in claim 1, wherein
the fringe-encoded image further comprises a second fringe group adjacent to the first fringe group;
the imaging plane comprises a second imaging interval adjacent to the first imaging interval;
in a case that the to-be-scanned object is located within the effective depth-of-field range of the three-dimensional scanning device, an image of the second fringe group on the imaging plane is located within the second imaging interval, and only the second fringe group exists in the second imaging interval.

5. The three-dimensional scanning device as claimed in claim 4, wherein
the fringe-encoded image comprises a plurality of fringe groups periodically arranged, and the first fringe groups and the second fringe groups are respectively located in a cycle.

6. The three-dimensional scanning device as claimed in claim 4, wherein
a system included angle α is formed between a projection optical axis of the projection device and a collection optical axis of the camera, optical parameters of the camera comprise lens magnification k₁, an effective depth of field ΔL of the three-dimensional scanning device and the second imaging interval d₂, and d₂=ΔL×tgα÷k₁.

7. The three-dimensional scanning device as claimed in claim 4, wherein
the projection device comprises an image display element, the image display element comprising a second display interval provided with the second fringe group; and
optical parameters of the projection device comprise lens magnification k₂ of the projection device and the second display interval D₂, and D₂=ΔL×tgα÷k₂.

8. The three-dimensional scanning device as claimed in claim 1, the three-dimensional scanning device further comprises:
a processor, configured to perform three-dimensional reconstruction on the to-be-scanned object based on the camera image.

9. The three-dimensional scanning device as claimed in claim 8, wherein
first imaging interval coordinates are preset in the processor;
the processor determines, based on the camera image, pixel coordinates of a center of each fringe in the camera image;
the processor determines, based on the pixel coordinates of the fringes and the first imaging interval coordinates, a number of each fringe in the camera image; and
the processor performs, based on the pixel coordinates of the center of each fringe and the number of each fringe, three-dimensional reconstruction to obtain a three-dimensional digital model of the to-be-scanned object.

10. The three-dimensional scanning device as claimed in claim 8, wherein
a light plane of each fringe and a corresponding number thereof in the fringe-encoded image are preset in the processor;
the processor determines, based on consistency between a number of each fringe in the camera image and the corresponding number of the light plane of each fringe, a light plane corresponding to the pixel coordinates of the center of each fringe; and
the processor performs, based on the pixel coordinates of the center of each fringe and the corresponding light plane, trigonometric calculation to reconstruct a three-dimensional digital model of the to-be-scanned object.

11. A three-dimensional scanning method, executed based on the three-dimensional scanning device as claimed in claims 1-7, comprises:
projecting a fringe-encoded image to a to-be-scanned object through a projection device;
collecting the to-be-scanned object by a camera to obtain a camera image, wherein the camera image is an image of the to-be-scanned object on an imaging plane of the camera, the imaging plane comprises a first imaging interval, and in a case that the to-be-scanned object is located within an effective depth-of-field range of the three-dimensional scanning device, the image of the first fringe group on the imaging plane is located within the first imaging interval, and only the first fringe group exists in the first imaging interval; and
performing, by a processor, three-dimensional reconstruction on the to-be-scanned object based on the camera image.

12. The three-dimensional scanning method as claimed in claim 11, the three-dimensional scanning method further comprises:
determining, based on the camera image, pixel coordinates of a center of each fringe in the camera image;
presetting first imaging interval coordinates in the processor, and determining a number of each fringe based on the pixel coordinates of the fringes and the first imaging interval coordinates; and
performing three-dimensional reconstruction on the pixel coordinates of the center of each fringe based on the numbers to obtain a three-dimensional digital model of the to-be-scanned object.

13. A three-dimensional scanning method, comprising:
projecting a fringe-encoded image to a to-be-scanned object, wherein the fringe-encoded image comprises a time-encoded image or color-encoded image, the time-encoded image comprises a plurality of time fringe patterns arranged based on time, and the color-encoded image comprises a color fringe pattern encoded by a plurality of colors;
collecting a three-dimensional reconstructed image of the to-be-scanned object, wherein a surface of the to-be-scanned object in the three-dimensional reconstructed image has the fringe-encoded image; and
reconstructing, based on the three-dimensional reconstructed image, a three-dimensional model of the to-be-scanned object.

14. The three-dimensional scanning method as claimed in claim 13, wherein in a case that the fringe-encoded image is the time-encoded image, the three-dimensional scanning method comprises:
projecting a first time fringe pattern to the surface of the to-be-scanned object at a first time;
obtaining a first time fringe image on the surface of the to-be-scanned object;
projecting a second time fringe pattern to the surface of the to-be-scanned object at a second time;
obtaining a second time fringe image on the surface of the to-be-scanned object; and
determining a time image encoding table based on the first time fringe image and the second time fringe image.

15. The three-dimensional scanning method as claimed in claim 14, wherein determining the time image encoding table based on the first time fringe image and the second time fringe image comprises:
determining a first encoding table based on the first time fringe image;
determining a second encoding table based on the second time fringe image; and
constructing the time image encoding table based on the first encoding table and the second encoding table.

16. The three-dimensional scanning method as claimed in claim 15, wherein
determining the first encoding table based on the first time fringe image comprises:
correspondingly assigning first encoded values to pixels with fringes in the first time fringe image, correspondingly assigning second encoded values to pixels without fringes in the first time fringe image, and constructing the first encoding table by the first encoded values and the second encoded values based on pixel position distribution of the first time fringe image;
determining the second encoding table based on the second time fringe image comprises:
correspondingly assigning first encoded values to pixels with fringes in the second time fringe image, correspondingly assigning second encoded values to pixels without fringes in the second time fringe image, and constructing the second encoding table by the first encoded values and the second encoded values based on pixel position distribution of the second time fringe image; and
constructing the time image encoding table based on the first encoding table and the second encoding table comprises:
arranging encoded values at same pixel positions in the first encoding table and the second encoding table according to an obtaining sequence of the first time fringe image and the second time fringe image to serve as encoding sequences of corresponding pixels, and constituting the time image encoding table based on the encoding sequences.

17. The three-dimensional scanning method as claimed in claim 14, wherein after obtaining the second time fringe image on the surface of the to-be-scanned object, the three-dimensional scanning method further comprises:
projecting a third time fringe pattern to the surface of the to-be-scanned object at a third time;
obtaining a third time fringe image on the surface of the to-be-scanned object; and
determining a time image encoding table based on the first time fringe image, the second time fringe image and the third time fringe image.

18. The three-dimensional scanning method as claimed in claim 17, wherein determining the time image encoding table based on the first time fringe image, the second time fringe image and the third time fringe image comprises:
correspondingly assigning first encoded values to pixels with fringes in the first time fringe image, correspondingly assigning second encoded values to pixels without fringes in the first time fringe image, and constructing a first encoding table by the first encoded values and the second encoded values based on pixel position distribution of the first time fringe image;
correspondingly assigning first encoded values to pixels with fringes in the second time fringe image, correspondingly assigning second encoded values to pixels without fringes in the second time fringe image, and constructing a second encoding table by the first encoded values and the second encoded values based on pixel position distribution of the second time fringe image;
correspondingly assigning first encoded values to pixels with fringes in the third time fringe image, correspondingly assigning second encoded values to pixels without fringes in the third time fringe image, and constructing a third encoding table by the first encoded values and the second encoded values based on pixel position distribution of the third time fringe image; and
arranging encoded values at same pixel positions in the first encoding table, the second encoding table and the third encoding table according to an obtaining sequence of the first time fringe image, the second time fringe image and the third time fringe image to serve as encoding sequences of corresponding pixels, and constituting the time image encoding table based on the encoding sequences.

19. The three-dimensional scanning method as claimed in claims 13-18, wherein encoding table adopts binary encoding, and encoded values corresponding to pixels with fringes in the time-encoded image are denoted by 1, and encoded values corresponding to pixels without fringes in the time-encoded image are denoted by 0.

20. The three-dimensional scanning method as claimed in claim 14, wherein after determining the time image encoding table based on the first time fringe image and the second time fringe image, the three-dimensional scanning method further comprises:
projecting a fourth time fringe pattern to the surface of the to-be-scanned object to obtain a fourth time fringe image on the surface of the to-be-scanned object, and determining a sequence of each fringe in the fourth time fringe image based on the time image encoding table; and
projecting a fifth time fringe pattern to the surface of the to-be-scanned object to obtain a fifth time fringe image on the surface of the to-be-scanned object, and determining a sequence of each fringe in the fifth time fringe image based on the time image encoding table, wherein the fifth time fringe pattern is obtained by deflecting fringes in the fourth time fringe pattern by a distance d in a same direction.

21. The three-dimensional scanning method as claimed in claim 13, wherein in a case that the fringe-encoded image is the color-encoded image, the three-dimensional scanning method comprises:
projecting the color-encoded image to the surface of the to-be-scanned object, the color-encoded image comprising a first color fringe pattern and a second color fringe pattern;
obtaining color fringe images on the surface of the to-be-scanned object, the color fringe images comprising a first color fringe image and a second color fringe image; and
determining a color image encoding table based on the first color fringe image and the second color fringe image.

22. The three-dimensional scanning method as claimed in claim 21, wherein determining the color image encoding table based on the color fringe images, comprising:
determining a first color encoding table based on the first color fringe image;
determining a second color encoding table based on the second color fringe image;
constructing the color image encoding table based on the first color encoding table and the second color encoding table.

23. The three-dimensional scanning method as claimed in claim 22, wherein
determining the first color encoding table based on the first color fringe image comprises:
correspondingly assigning a first encoding sequence to pixels with a first color in the first color fringe image, correspondingly assigning a fourth encoding sequence to pixels without the first color in the first color fringe image, and constructing the first color encoding table by the first encoding sequence and the fourth encoding sequence based on pixel position distribution of the first color fringe image;
determining the second color encoding table based on the second color fringe image comprises:
correspondingly assigning a second encoding sequence to pixels with a second color in the second color fringe image, correspondingly assigning a fourth encoding sequence to pixels without the second color in the second color fringe image, and constructing the second color encoding table by the second encoding sequence and the fourth encoded sequence based on pixel position distribution of the second color fringe image;
constructing the color image encoding table based on the first color encoding table and the second color encoding table comprises:
superposing encoding sequence at same pixel positions in the first color encoding table and the second color encoding table as encoding sequences of corresponding pixels, and constituting the color image encoding table based on superimposed encoding sequences corresponding to a distribution of each pixel.

24. The three-dimensional scanning method as claimed in claims 21-23, wherein encoding table adopts binary encoding, and a first encoding sequence corresponding to pixels with a first color in the color-encoded image is (0,0,1), and a second encoding sequence corresponding to pixels with a second color in the color-encoded image is (0,1,0), and a fourth encoding sequence corresponding to pixels without color in the color-encoded image is (0,0,0).

25. A three-dimensional scanning apparatus, comprising:
a projection unit, configured to project a fringe-encoded image to a to-be-scanned object, wherein the fringe-encoded image includes a time-encoded image or color-encoded image, the time-encoded image includes a plurality of time fringe patterns arranged based on time, and the color-encoded image includes a color fringe pattern encoded by a plurality of colors;
an acquisition unit, configured to collect a three-dimensional reconstructed image of the to-be-scanned object, wherein a surface of the to-be-scanned object in the three-dimensional reconstructed image has the fringe-encoded image; and
a reconstruction unit, configured to reconstruct, based on the three-dimensional reconstructed image, a three-dimensional model of the to-be-scanned object.

26. A computer-readable storage medium or a non-transitory storage medium, wherein the computer-readable storage medium or the non-transitory storage medium includes stored programs, the programs, when running, control a device that the computer-readable storage medium or the non-transitory storage medium is located to execute the three-dimensional scanning method as claimed in any one of claims 13 to 24.

27. A processor, wherein the processor is configured to run programs that, when executed by the processor, cause the processor to execute the three-dimensional scanning method as claimed in any one of claims 13 to 24.
